# EUROPEAN PATENT APPLICATION

(11) **EP 2 213 304 A1**
(43) Date of publication of application: **04.08.2010**
(21) Application number: 08835311.5
(22) Date of filing: 03.10.2008
(51) Int. Cl.: A61K 47/02, A23L 1/00, A61K 9/16, A61K 9/20, A61K 31/015, A61K 31/047, A61K 31/07, A61K 31/19, A61K 31/202, A61K 31/22, A61K 31/23, A61K 31/353, A61K 31/355, A61K 31/381, A61K 36/60, A61K 36/73, A61K 47/26, A61K 47/36

(54) **GRANULE, TABLET, AND THEIR PRODUCTION METHODS**

(30) Priority: 03.10.2007 JP 2007260026; 21.12.2007 JP 2007330050
(71) Applicant: ASAHI BREWERIES, Ltd., Sumida-ku Tokyo 130-8602 (JP); Tokuyama Corporation, Shunan-shi, Yamaguchi 745-8648 (JP)
(72) Inventor: HIRAI, Nobuaki, Ibaraki 302-0106 (JP); ISHIKIKAWA, Kazuyuki, Ibaraki (JP); AZECHI, Yasutaka, Ibaraki (JP); ETOU, Yoshitaka, Sumida-ku, Tokyo (JP)
(74) Representative: Müller, Christian Stefan Gerd
(86) International application number: PCT/JP2008/068051
(87) International publication number: WO 2009/044854

(57) **Abstract**

A granule comprising a carrier composed of porous calcium silicate; a functional substance which is an oily liquid or a low-melting solid and a water-soluble antioxidizing agent, which are adsorbed on the carrier; and a granulating component. The problem to be solved is to improve storage stability of a powdered or tableted functional substance, for example, vitamin A to the level causing no problem in practical use.

## Description

### Technical Field

The present invention relates to a granule and a tablet and methods for producing the same, particularly to a granule and a tablet comprising an oily liquid or a low-melting solid as a functional substance and methods for producing the same.

### Background Art

Dosage forms for ingestion of a functional substance, which is an active ingredient of pharmaceuticals and health food, include powders, granules, capsules, tablets and the like. Among them, tablets are easy to handle and ingest and most frequently used.

Tablets are generally produced by-adding water and/or an organic solvent to a powder mixture combining a functional substance and optional components such as excipients, binders and disintegrating agents (all of which are polymeric additives derived from cellulose or natural or synthetic polymers) to perform granulation; putting the granulated granules into a turntable of a tableting machine from a hopper (a vessel containing the granulated granules) quantitatively; and pressing the granulated granules, which have been put in a hole (mortar) formed on the turntable, with 2 pairs of upper and lower steel pestles (upper pestle and lower pestle).

Since tablets are formed by pressing as described above, when a functional substance is oily liquid or low-melting solid in ambient temperature environment, it is difficult to form the functional substance into tablets as it is. In the case when the functional substance has such forms, the functional substance is subjected to treatment that changes these forms into solid or powder.

Japanese Patent Laid-Open Publication No. Sho 63(1988)-243034 describes a technique in which a fat-soluble drug such as vitamin E or vitamin E nicotinate ester or a low-melting substance such as Menfegol or natural extract as a functional substance is adsorbed on a carrier for immobilization. As the carrier, soft anhydrous silicic acid, calcium silicate and the like are employed. The supported material, a material having the functional substance adsorbed on the carrier, is then granulated using methylcellulose, and formed into tablet using a rotary tableting machine.

WO 2007/97333 describes that calcium silicate is employed as a carrier for adsorbing and powderizing a functional substance which is an oily liquid or a low-melting solid, and starch or sugar is employed as a granulating component. As a result, loose bulk density of the resulting powder is appropriately controlled, and it becomes possible to form thickly a tablet using a tableting machine. Accordingly, the tablet which employs calcium silicate as a carrier is provided with appropriate diameter and thickness and an appropriate weight to stand practical use.

The problems in terms of formability and shape (shaping ability) of the tablet which employs calcium silicate as a carrier have been solved as described above for the practical use. The problem that the functional substance which has been powdered or tableted tends to be deteriorated, and poor in storage stability has become newly evident, however.

Japanese Patent Laid-Open Publication No. Hei 7(1995)-285876 describes that polyphenol has functions such as antioxidative property. Polyphenol has also attracted attention as a substance with health-promoting benefits on human body. Polyphenol has to be uniformly in contact with an object substance, and it is preferably dissolved or finely dispersed in order to exert antioxidative function. Polyphenol is hydrophilic, however, and cannot be dispersed homogeneously in lipophilic substances (separated in two layers) as it is, and is difficult to function as an antioxidizing agent for lipophilic substances.

Vitamin A, for example, is a fat-soluble vitamin important in biochemistry, medication and pharmacy, and it has been highly demanded also as health food. The vitamin A is readily oxidized, however, and extremely unstable in environment where it is exposed to air. In order to maintain vitamin A stable, processes such as esterification, addition of antioxidizing agents, cool preservation with blocking air have to be conducted.

Examples of stabilizing substances employed in combination with vitamin A and its derivatives include fats and oils resistant to oxidization (Japanese Patent Laid-Open Publication No. 2001-97858), egg albumin or wool keratin (Japanese Patent Laid-Open Publication No. 2007-15970) and amino acids and fatty acid esters of polyhydric alcohols (Japanese Patent Laid-Open Publication No. Hei 6(1994)-32774).

Vitamin A combined with these stabilizing substances is in a liquid or semi-solid form, however, and inconvenient for handling and ingestion. Japanese Patent Laid-Open Publication No. 2007-15970 suggests formulation into soft capsule, but labor and cost are required for fabricating into a soft capsule.

### Disclosure of Invention

### Problems to be Solved by the Invention

The present invention is made for solving the conventional problems, and an objective thereof is to improve storage stability of a powdered or tableted functional substance, for example, vitamin A to the level causing no problem in practical use.

### Means for Solving the Problems

The present invention provides a granule comprising a carrier composed of porous calcium silicate, a functional substance which is an oily liquid or a low-melting solid and a water-soluble antioxidizing agent, which are adsorbed on the carrier, and a granulating component.

The present invention provides a granule comprising porous calcium silicate, a vitamin A-containing liquid which is obtained by dissolving or dispersing vitamin A or a vitamin A derivative in an oily liquid or a low-melting solid, a water-soluble antioxidizing agent, and a granulating component.

The present invention provides a method for producing a granule comprising the steps of:
adsorbing a functional substance and a water-soluble antioxidizing agent on a carrier composed of porous calcium silicate; and
mixing the resulting supported material with a granulating component to perform granulation.

The present invention provides a method for producing a granule comprising the steps of:
dissolving or dispersing vitamin A or a vitamin A derivative in an oily liquid or a low-melting solid to obtain a vitamin A-containing liquid;
mixing the vitamin A-containing liquid and porous calcium silicate; and
mixing the resulting mixture, a granulating component and a water-soluble antioxidizing agent.

The present invention provides a method for producing a tablet comprising the steps of:
charging the granule described above into a cylindrical mortar; and
compressing the granule filled in the mortar with a pestle.

The present invention provides a tablet obtainable by the method described above.

### Advantages of the Invention

The storage stability of a powdered or tableted functional substance, for example, vitamin A is improved to the level causing no problem in practical use. In addition, it became possible for a water-soluble antioxidizing agent to exert antioxidative function against a lipophilic substance.

### Best Embodiment for Carrying Out the Invention

### Granule

A granule of the present invention contains a carrier, a functional substance, a water-soluble antioxidizing agent, and a granulating component. At least the functional substance and the water-soluble antioxidizing agent are adsorbed on the carrier.

The carrier means a solid substance which can support a liquid for immobilization. Fine powders or porous materials having excellent chemical stability are generally employed as the carrier. Specific examples of the carrier include fine powders of silicic acid and of metal salts of silicic acid. Porous silica is described in, for example, Japanese Patent Laid-Open Publication Nos. Hei 6(1994)-40714 and Hei 8(1996)-157362, and it may be employed as a carrier on which a liquid is supported for immobilization.

Specific examples of the metal salts of silicic acid include calcium silicate, magnesium silicate, aluminum calcium silicate and the like. The carrier preferred for the granule of the present invention is porous calcium silicate. The porous calcium silicate is excellent in oil-absorption property, and has high immobilization ability for liquid. The porous calcium silicate also has a high ability of dividing liquid into small portions and therefore, when the functional substance and the water-soluble antioxidizing agent are adsorbed thereon, it allows them to be divided into small portions and coexist in a highly dispersed manner. As a result, even if the functional substance is a lipophilic substance, the functional substance uniformly contacts with the water-soluble antioxidizing agent, and antioxidative function of the water-soluble antioxidizing agent is effectively exerted.

Particularly preferred porous calcium silicate has an average particle diameter of 18 to 32 µm, a loose bulk density of 0.07 to 0.15 g/ml, and an oil absorption of 300 to 550 ml/100g. The calcium silicate powder having these properties has the following composition:

[Formula 1] 2CaO·3SiO₂·mSiO₂·nH₂O

wherein 1<m<2, and 2<n<3, and

is exemplified by gyrolite-type calcium silicate powder having a petal-shaped crystalline structure that is observed by an electronic microscope. Specifically, trade name "FLORITE" of Tokuyama Corporation is mentioned.

The carrier is combined in a ratio of 20 to 200 parts by weight, preferably 50 to 150 parts by weight based on 100 parts by weight of the functional substance. When the amount of the carrier is less than 20 parts by weight, the functional substance tends to leach out from the carrier, and problems of capping or sticking during tableting the granules or of hardness deficiency or poor appearance of tablet occur. When the amount of the carrier exceeds 200 parts by weight, light granules are produced so that it is difficult to fill a predetermined amount of the granules in a mortar, and only very thin tablets with poor practical usefulness is provided even after tableting.

The functional substance means a substance which exerts an intended effect in a human body after ingested, and this concept encompasses active ingredients for pharmaceuticals and health food. The functional substance is not limited in its form so long as it can be adsorbed on a carrier by any method, but preferred forms are oily liquid and low-melting solid. This is because the functional substance having such a form tends to be deteriorated when formed into powder or tablet. The liquid means a substance which shows fluidity at ambient temperature (20°C), and the solid means a substance which does not show fluidity at ambient temperature. The low-melting means having a melting point of not more than 100 to 120°C.

Specific examples of the oily liquid include vitamin A, vitamin A derivatives, vitamin E, docosahexaenoic acid, eicosapentaenoic acid and the like. An oily liquid derived from natural products is a substance extracted from the natural products, and generally means oily extracts mainly composed of oil-soluble components and substances which cannot be dried due to a high content of organic acids such as acetic acid that are uncrystallizable. Specific examples of the oily liquid derived from natural products include saw palmetto extract, products obtained by lactic fermentation of vegetable juice, black vinegar and the like.

The low-melting solid causes sticking phenomenon when compressed at ambient temperature, and is thus difficult to be compressed for shaping like the oily liquid. The low-melting solid is therefore also useful as the functional substance of the present invention. Specific examples of the low-melting solid include Coenzyme Q-10, alpha lipoic acid, ribose and the like.

As described above, using the porous calcium silicate as a carrier for immobilizing a functional substance, it became possible for the water-soluble antioxidizing agent to exert its antioxidative function sufficiently in combination with a lipophilic substance. No surface active agent such as an emulsifying agent and a dispersing agent has to be employed for that case. Examples of particularly preferred lipophilic functional substance used in combination with porous calcium silicate and a water-soluble antioxidizing agent include, for example, vitamin A, vitamin A derivatives, vitamin E, vitamin D, vitamin K, docosahexaenoic acid, eicosapentaenoic acid, beta-carotene, lutein, astaxanthin, lycopene, lipoic acid, linoleic acid, gamma-linoleic acid, tocotrienol, evening primrose oil, saw palmetto extract and products obtained by lactic fermentation of vegetable juice, more preferably, vitamin A, vitamin A derivatives, vitamin E, docosahexaenoic acid, eicosapentaenoic acid, lipoic acid, evening primrose oil, beta-carotene and saw palmetto extract.

These may be dissolved or dispersed in the oily liquid or the low-melting solid to prepare a functional substance-containing liquid, and employed. A vitamin A-containing liquid which is combined with the stabilizing substances described in Japanese Patent Laid-Open Publication Nos. 2001-97858, 2007-15970 and Hei 6(1994)-32774 is particularly preferred as a lipophilic functional substance employed for the present invention.

Vitamin A is a fat-soluble vitamin highly contained in liver oil and fish oil and the like, and also referred to as retinol or axerophthol. As the derivative of vitamin A, acetate ester or palmitate ester of vitamin A may be exemplified. The vitamin A acetate ester is described in the Japanese Pharmacopoeia as retinol acetate, and the palmitate ester is usually available as vitamin A oil as described in the Japanese Pharmacopoeia. Vitamin A and vitamin A derivatives are together referred to as "vitamin A and analogs".

The oily liquid or the low-melting solid is not limited, so long as it dissolves or disperses the vitamin A and analogs and it is not easily oxidized under ordinary storage conditions such as ambient temperature and normal pressure. The liquid means a substance which shows fluidity at ambient temperature (20°C), and the solid means a substance which does not show fluidity at ambient temperature. The low-melting means having a melting point of not more than 100 to 120°C.

Preferred oily liquids or low-melting solids are fatty acid glycerides. This is because these are excellent in ability of dissolving vitamin A and analogs. Oils and fats means glycerin esters of fatty acids. The glycerin ester may be a monoester, a diester, a triester, or a mixture thereof.

A medium-chain fatty acid is preferred as the fatty acid. This is because oils and fats composed of medium-chain fatty acids, particularly medium-chain saturated fatty acids has high stability. The medium-chain fatty acid in the present invention means fatty acids, particularly saturated fatty acids, having 6 to 12 carbon atoms. Examples include caproic acid, caprylic acid, capric acid, lauric acid, and particularly preferred are saturated fatty acids having 8 to 10 carbon atoms, specifically caprylic acid and capric acid.

The oily liquid or the low-melting solid may be polyhydric alcohol esters of fatty acids, particularly medium-chain fatty acids, polyoxyethylene or polyoxypropylene adduct of polyhydric alcohol, liquid paraffin, vitamin E (tocopherol), and an acetate ester of vitamin E.

The polyhydric alcohol includes propylene glycol, trimethylolpropane and sorbitol. The acetate ester of vitamin E includes dl-alpha-tocopherol acetate, d-alpha-tocopherol acetate, 1-alpha-tocopherol acetate and the like.

The oily liquid or the low-melting solid is combined in a ratio of 50 to 1200 parts by weight, preferably 60 to 1000 parts by weight, more preferably 100 to 400 parts by weight based on 100 parts by weight of the vitamin A and analogs. When the amount of the oily liquid or the low-melting solid is less than 50 parts by weight, storage stability of the vitamin A and analogs is deteriorated. When the amount exceeds 1200 parts by weight, powderization becomes difficult.

The water-soluble antioxidizing agent is not limited, so long as it is a substance conventionally employed as food additives. Preferred examples thereof is, for example, polyphenol, vitamin C (ascorbic acid) and salts thereof, erythorbic acid and salts thereof, sorbic acid and salts thereof, rosemary extracts, catechin and the like. They may be employed alone or in combination of more than two.

It is preferred that the polyphenol is derived from a plant. Particularly, polyphenols derived from fruits of apple, peach and pear, hop and tea are preferred, and apple polyphenol and hop polyphenol are most preferred.

The apple polyphenol prepared by the method described in Japanese Patent Laid-Open Publication No. Hei 7(1995)-285876 can be used. The apple polyphenol is composed of polyphenol fractions purified from an extract or a squeezed juice of apple fruits, preferably immature fruits.

The fruit squeeze method includes a method comprising washing and crushing raw material, and compressing it as it is or with addition of sulfurous acid to obtain squeezed juice, and preferably adding pectinolytic enzyme thereto. The resultant is then subjected to procedures such as centrifugal separation and filtration to obtain clear fruit juice.

The extraction method includes a method comprising mixing washed raw material with alcohol (ethanol, methanol and the like) and crushing, extracting by dipping as it is, compressing, or heating to reflux, and after distilling alcohols off by vacuum concentration, subjecting the resultant to centrifugation separation and filtration, or to partition with organic solvents (hexane, chloroform and the like) and filtration to obtain clear extract liquid.

The purification method includes a method comprising passing clear juice or clear extract liquid through a column filled with an adsorbent which can selectively adsorb and elute polyphenol, for example, a styrenedivinylbenzene type synthetic adsorbent resin, an anion exchange resin, an octadecyl group chemically bonded silica gel (ODS), to adsorb polyphenol fractions. After distilled water is then passed through the column to wash, and 20 to 100% alcohol (e.g., ethanol) solution, preferably about 50% alcohol solution is passed through the column so that the polyphenol fraction can be eluted and recovered.

The resulting polyphenol solution is concentrated under vacuum to distil alcohol off so that an apple polyphenol liquid preparation (preferably containing an organic acid such as malic acid added) can be obtained. The liquid preparation is spray dried or freeze dried as it is or with addition of a powder aid such as dextrin to obtain an apple polyphenol powder preparation.

The apple polyphenol contains as components, for example, simple polyphenol compounds such as caffeic acid derivatives, p-coumaric acid derivatives, flavan-3-ols (catechins), flavonols (quercetin glycosides), dihydrochalcones (phloretin glycosides) and the like, and polymer polyphenol compounds such as condensed tannins and the like.

The hop polyphenol prepared by the method described in Japanese Patent Laid-Open Publication Nos. Hei 9(1997)-295944 and Hei 9(1997)-2917 can be used. The hop polyphenol is obtained by extracting a water-soluble fraction from hop or hop bract, passing it through a gel type synthetic adsorbent, washing with water or aqueous ethanol, and eluting with ethanol or aqueous ethanol. The hop polyphenol contains as components, for example, catechins, and flavonoids such as rutin and isoquercitrin. Preferred polyphenol includes catechin, procyanidin derived from apple and the like.

In an embodiment, the polyphenol is combined in a ratio of preferably 3 to 20 parts by weight, more preferably 5 to 10 parts by weight based on 100 parts by weight of the functional substance. When the amount of the polyphenol is less than 3 parts by weight, storage stability of the functional substance formed into powder or tablets becomes insufficient, and when the amount exceeds 20 parts by weight, cost is increased, and it becomes impossible to produce tablets inexpensively.

In another embodiment, the water-soluble antioxidizing agent is combined in a ratio of 5 to 800 parts by weight, preferably 10 to 600 parts by weight, more preferably 20 to 100 parts by weight based on 100 parts by weight of the vitamin A and analogs. When the amount of the antioxidizing agent is less than 5 parts by weight, storage stability of the vitamin A and analogs becomes insufficient, and when the amount exceeds than 800 parts by weight the granule or the tablet becomes hygroscopic, and a cost is increased and it becomes impossible to produce tablets inexpensively.

The granulating component means a component which is employed for binding carrier particles to form granules. The granulating component is not limited so long as it is material exerting a function to bind particle. Generally, starch, povidone, dextrin, hydroxypropylcellulose, hydroxypropylmethylcellulose, methylcellulose, polyvinyl alcohol, sodium carboxymethylcellulose, alpha starch, sodium alginate, pullulan, gum arabic powder and the like are employed.

Preferred granulating components are starches, sugars or mixture of starches and sugars. For example, when calcium silicate alone is employed as the carrier, calcium silicate is light and thus granulation is difficult. However, a granule having an appropriate loose bulk density and particle size distribution can be obtained by adding the starches, the sugars or the mixtures of starches and sugars and performing granulation.

When the starch or the sugar is present, it is easy to provide a dense state condition to reduce a contact area between oil and air, and thus antioxidative function of the polyphenol can be drawn out maximally.

Any known starch can be employed, and examples thereof include wheat starch, rice starch, maize starch, potato starch, and tapioca starch. The examples further include dextrin and the like obtainable by enzymatic degradation, acid degradation, or alkaline degradation of these starches, or by performing these reactions in combination.

Preferred starches are dextrins. Examples of dextrins include maltodextrin, achrodextrin, erythrodextrin, and amylodextrin, and any of these can be employed. A preferred dextrin is maltodextrin.

Any known sugar can be employed, and examples thereof include glucose and maltose. Sugar alcohols obtainable by hydrogenation of these sugars may also be employed. Sugar alcohols are preferred. Examples of sugar alcohols include sorbitol, erythritol, xylitol, and maltitol, and any of these can be employed.

The amount of the granulating component employed is not particularly limited. In general, when too small an amount is employed, a granule becomes light. When too large an amount is employed, the content of the functional substance is decreased, and a large amount of granules are required for tableting. These amounts are not practical. The granulating component is combined in a ratio of 20 to 500 parts by weight, preferably 30 to 300 parts by weight, more preferably 50 to 200 parts by weight based on 100 parts by weight of a carrier. When the amount of the granulating component is less than 20 parts by weight, the loose bulk density of granules becomes too low, and thereby a tablet can hardly be made thick. When the amount of the granulating component exceeds 500 parts by weight, only a small amount of the functional substance can be combined, and thereby the concentration of the functional substance contained in the tablet becomes too low.

In addition to a carrier, a functional substance, a water-soluble antioxidizing agent, and a granulating component, the granule of the present invention may contain additives such as a lubricant and a disintegrating agent as required. For example, lubricants such as hydrogenated oil, hydrogenated castor oil, stearic acid, magnesium stearate, calcium stearate, glyceride behenate, sodium stearyl fumarate, and sucrose fatty acid esters and disintegrating agents such as low-substituted hydroxypropylcellulose, carmellose, carboxymethyl starch sodium, crospovidone, croscarmellose sodium, starches, and agar may be combined.

When calcium silicate alone is employed as a carrier in the granule of the present invention, the content of the functional substance can be significantly increased, with the content of the functional substance being 10 to 50% by weight, preferably 25 to 35% by weight. Furthermore, when a starch, a sugar, or a mixture of a starch and a sugar is employed as a granulating component in the granule of the present invention, the loose bulk density is 0.20 to 0.80 g/ml, preferably 0.30 to 0.60 g/ml.

By adjusting the loose bulk density of the granule to this range, a tablet having appropriate diameter, thickness, and weight can be obtained using a usual tableting machine. That is, a tablet having a strength that is sufficient for practical use and a shape and a size that are convenient for handling can be provided using the granule of the present invention.

Coating using a water-soluble polymer may be applied to the granule of the present invention, if necessary. By applying the coating, an odor and a taste derived from a water-soluble antioxidizing component can be reduced. Furthermore, the property for ingestion and the moisture resistance of the granule are improved. Methods and materials to be employed for granule coating in the present invention are not limited. Examples of water-soluble polymers generally include hydroxypropylcellulose, hydroxypropylmethylcellulose, polyvinylpyrrolidone, and methylcellulose. Examples of water-insoluble polymers include shellac, zein, ethylcellulose, and hydroxypropylmethylcellulose phthalate.

### Method for producing granules

A solution containing a functional substance is prepared by a usual method or a method described in Japanese Patent Laid-Open Publication Nos. 2001-97858, 2007-15970, or Hei 6(1994)-32774, or the like by dissolving or dispersing the functional substance in an oily liquid or a low-melting solid, or the like.

A functional substance is adsorbed on and supported with a carrier by dissolving or dispersing the functional substance in a solvent to decrease viscosity as required; mixing the mixture with a carrier, and suitably drying the product. As a result, the functional substance is held tightly in a fine pore of the carrier, the functional substance does not leach out to the granule surface during tableting, a mold-compressing property is improved, and a sticking phenomenon or the like is prevented.

A preferred organic solvent for decreasing viscosity of the functional substance is ethanol because of high safety thereof. Furthermore, a small amount of water may be added together with ethanol. An organic solvent in which the functional substance is dissolved or dispersed and a carrier may be mixed in any vessel, but usually a blender, a stirring granulator employed during the granulation step described later, or the like can be employed with stirring.

The amount of the organic solvent employed in which the functional substance is dissolved or dispersed is sufficient so long as viscosity is decreased by dissolving or dispersing the functional substance to be dissolved. When the amount of the organic solvent is too small, the functional substance is not uniformly adsorbed, and when the amount is too large, the supported material becomes wet. The amount can be suitably adjusted.

To allow a water-soluble antioxidizing agent to be adsorbed on and supported by a carrier, a water-soluble antioxidizing agent or an aqueous solution thereof is mixed with a carrier. This step is preferably performed after adsorption of the functional substance on a carrier. This is because positioning a functional substance and a water-soluble antioxidizing agent in this order from the depth in a fine pore of the carrier is effective for obtaining an antioxidizing effect.

However, the step of adsorbing a water-soluble antioxidizing agent on a carrier may be performed before or at the same time as the step of adsorbing a functional substance. When these steps are performed simultaneously, a water-soluble antioxidizing agent may be dissolved or dispersed in an organic solvent together with a functional substance and absorbed on the carrier.

A supported material obtainable by absorbing the functional substance or the like in a carrier usually remains powdery. However, when the supported material becomes wet, the supported material is suitably dried for powderization.

Subsequently, the resulting supported material (a mixture of a carrier and a functional substance or a solution containing the same) is granulated or made granular. The supported material is granulated by a usual method using a granulating component, water, and an organic solvent. The water-soluble antioxidizing agent may be added in a powder or by dissolving in water for granulation beforehand when or after a granulating component is added to the supported material. In general, the granulating component is water-soluble. So, even when the granulating component is added in a powder, it is dissolved together with the water-soluble antioxidizing agent, and uniformly mixed in the supported material, and the mixture is adsorbed so that a fine pore of porous calcium silicate is blocked.

For granulation, either spraying granulation or stirring granulation can be performed, but stirring granulation is preferred because granules with a high bulk density can be obtained. The stirring granulators are in various forms, including those having a rotating arm above a vessel, those having a rotating wing at the bottom of a vessel, and further those to which stirring in different directions is applied.

Examples of the apparatus employed for granulation include vertical granulators, high-share mixers, high-speed mixers, planetary mixers, Collette Gral granulators, and chopper-type planetary mixers. It is sufficient to use an apparatus that can appropriately mix a powder for granulation. An apparatus with a stronger stirring power progresses granulation faster.

The granule thus obtained is dried, if necessary, to remove water or an organic solvent contained. Drying temperature is 20 to 90°C, preferably 25 to 75°C, more preferably 30 to 60°C.

### Method for producing tablet

The dried granule is sized, polymer additives and a lubricant are added, and the mixture is compressed in a mold to produce tablets. Polymer additives generally employed to produce granules or tablets can be employed. Specific examples thereof include celluloses such as crystalline cellulose, hydroxypropylcellulose, and hydroxypropylmethylcellulose, synthetic polymers such as polyvinylpyrrolidone, and natural polymers such as gum arabic, agar, and pullulan.

One or more polymer additives are employed in combination. It is recommended to use an excipient, a binder, and a disintegrating agent separately depending on the purpose. A coating step may be suitably added in which film coating or sugar coating is applied to thus produced tablets.

Tablets are produced by filling a powder containing granules or the like into a cylindrical mortar and compressing the granules filled in the mortar with a pestle. Any known tableting machine can be employed.

The present invention will be explained more specifically with reference to the following examples. However, the scope of the present invention is not limited to these Examples.

### Examples

### Example 1

A saw palmetto extract, a liquid extract derived from natural product, was prepared. Furthermore, apple polyphenol (trade name "APPLEPHENONE SH" manufactured by Asahi Food & Healthcare Co., Ltd.) was used as polyphenols. Components of this apple polyphenol are shown in Table 1. The content of the components corresponding to polyphenols in the apple polyphenol is 95%.

**[Table 1]**

| Component | Content (%) |
|---|---|
| Procyanidin | 52 |
| Phenol carboxylic acid | 26 |
| Catechin | 9 |
| Flavonoid | 8 |
| Others | 5 |
| Total | 100 |

38 parts by weight of a saw palmetto extract was added to 45.6 parts by weight of ethanol to obtain a dispersion. 38 parts by weight of calcium silicate (trade name "FLORITE R" manufactured by Tokuyama Corporation) was passed through a sieve and filled in a vertical granulator (GRANULATOR manufactured by Powerex Corporation). The calcium silicate is porous calcium silicate having a loose bulk density of 0.1 g/ml and an oil absorption of 480 ml/100 g. Then, the saw palmetto extract dispersion and an aqueous solution obtainable by dissolving 7.6 parts by weight of apple polyphenol in 76 parts by weight of water were filled and mixed for 5 minutes. Then, 16.4 parts by weight of dextrin was added, and the mixture was subjected to stirring granulation for 5 minutes, sized, and dried to obtain a granule containing a saw palmetto extract.

The resulting granule was placed on a tray and allowed to stand at 70°C for 48 hours. The residual ratio of beta-sitosterol, an indicator component contained in the saw palmetto extract, in the heated granule was 100.0%.

### Example 2

A saw palmetto extract, a liquid extract derived from a natural product, was prepared. 38 parts by weight of the saw palmetto extract was added to 45.6 parts by weight of ethanol to obtain a dispersion. 38 parts by weight of calcium silicate (trade name "FLORITE R" manufactured by Tokuyama Corporation) was passed through a sieve and filled in a vertical granulator. Then, the saw palmetto extract dispersion and an aqueous solution obtainable by dissolving 3.8 parts by weight of apple polyphenol in 76 parts by weight of water were filled and mixed for 5 minutes. Then, 20.2 parts by weight of xylitol was added, and the mixture was subjected to stirring granulation for 5 minutes, sized, and dried. The resulting granule was placed on a tray and allowed to stand at 70°C.

After 24 hours and 48 hours of heating, the residual ratio of beta-sitosterol, an indicator component contained in the saw palmetto extract, in the heated granule was both 100.0%.

### Example 3

A saw palmetto extract, a liquid extract derived from a natural product, was prepared. 38 parts by weight of the saw palmetto extract was added to 45.6 parts by weight of ethanol to obtain a dispersion. 38 parts by weight of calcium silicate (trade name "FLORITE R" manufactured by Tokuyama Corporation) was passed through a sieve and filled in a vertical granulator. Then, the saw palmetto extract dispersion and an aqueous solution obtainable by dissolving 7.6 parts by weight of apple polyphenol in 76 parts by weight of water were filled and mixed for 5 minutes. Then, 16.4 parts by weight of xylitol was added, and the mixture was subjected to stirring granulation for 5 minutes, sized, and dried. The resulting granule was placed on a tray and allowed to stand at 70°C.

After 24 hours and 48 hours of heating, the residual ratio of beta-sitosterol, an indicator component contained in the saw palmetto extract, in the heated granule was both 100.0%.

### Comparative Example 1

A saw palmetto extract, a liquid extract derived from a natural product, was prepared. 38 parts by weight of the saw palmetto extract was added to 22.9 parts by weight of ethanol to obtain a dispersion. 38 parts by weight of calcium silicate (trade name "FLORITE R" manufactured by Tokuyama Corporation) was passed through a sieve and filled in a vertical granulator. Then, the saw palmetto extract dispersion and 76 parts by weight of water were filled and mixed for 5 minutes. Then, 24 parts by weight of xylitol was added, and the mixture was subjected to stirring granulation for 5 minutes, sized, and dried. The resulting granule was placed on a tray and allowed to stand at 70°C.

The residual ratio of beta-sitosterol, an indicator component contained in the saw palmetto extract, in the heated granule was 98.3% after 24 hours of heating and 91.4% after 48 hours of heating.

### Comparative Example 2

A saw palmetto extract, a liquid extract derived from a natural product, was placed on a tray and allowed to stand at 70°C for 48 hours. The residual ratio of beta-sitosterol, an indicator component contained in the heated saw palmetto extract was 92.5% after 24 hours of heating and 80.2% after 48 hours of heating.

### Example 4

Beta-carotene, an oily component, was prepared. 16.7 parts by weight of beta-carotene (30% oil suspension preparation) was added to 58 parts by weight of ethanol to obtain a dispersion. 57.5 parts by weight of calcium silicate (trade name "FLORITE R" manufactured by Tokuyama Corporation) was passed through a sieve and filled in a vertical granulator. Then, the beta-carotene dispersion and an aqueous solution obtainable by dissolving 1.7 parts by weight of apple polyphenol in 144 parts by weight of water were filled and mixed for 5 minutes. Then, 24.1 parts by weight of xylitol was added, and the mixture was subjected to stirring granulation for 5 minutes, sized, and dried. The resulting granule was placed on a tray and allowed to stand at 70°C for 48 hours. The residual ratio of beta-carotene contained in the heated granule was 99.2%.

### Example 5

Beta-carotene, an oily component, was prepared. 16.7 parts by weight of beta-carotene (30% oil suspension preparation) was added to 58 parts by weight of ethanol to obtain a dispersion. 57.5 parts by weight of calcium silicate (trade name "FLORITE R" manufactured by Tokuyama Corporation) was passed through a sieve and filled in a vertical granulator. Then, the beta-carotene dispersion and an aqueous solution obtainable by dissolving 3.3 parts by weight of apple polyphenol in 144 parts by weight of water were filled and mixed for 5 minutes. Then, 22.5 parts by weight of xylitol was added, and the mixture was subjected to stirring granulation for 5 minutes, sized, and dried. The resulting granule was placed on a tray and allowed to stand at 70°C for 48 hours. The residual ratio of beta-carotene contained in the heated granule was 99.4%.

### Comparative Example 3

Beta-carotene, an oily component, was prepared. 16.7 parts by weight of beta-carotene (30% oil suspension preparation) was added to 58 parts by weight of ethanol to obtain a dispersion. 57.5 parts by weight of calcium silicate (trade name "FLORITE R" manufactured by Tokuyama Corporation) was passed through a sieve and filled in a vertical granulator. Then, the beta-carotene dispersion and 144 parts by weight of water were filled and mixed for 5 minutes. Then, 25.8 parts by weight of xylitol was added, and the mixture was subjected to stirring granulation for 5 minutes, sized, and dried. The resulting granule was placed on a tray and allowed to stand at 70°C for 48 hours. The residual ratio of beta-carotene contained in the heated granule was 75.1%.

### Comparative Example 4

Beta-carotene (30% oil suspension preparation), an oily component, was placed on a tray and allowed to stand at 70°C for 48 hours. After heating, the residual ratio of beta-carotene was 67.0%.

### Example 6

Lutein, an oily component, was prepared. 25 parts by weight of lutein (20% oil suspension preparation) was added to 58 parts by weight of ethanol to obtain a dispersion. 57.5 parts by weight of calcium silicate (trade name "FLORITE R" manufactured by Tokuyama Corporation) was passed through a sieve and filled in a vertical granulator. Then, the beta-carotene dispersion and an aqueous solution obtainable by dissolving 2.5 parts by weight of apple polyphenol in 144 parts by weight of water were filled and mixed for 5 minutes. Then, 15 parts by weight of xylitol was added, and the mixture was subjected to stirring granulation for 5 minutes, sized, and dried. The resulting granule was placed on a tray and allowed to stand at 70°C for 48 hours. The residual ratio of lutein contained in the heated granule was 100.0%.

### Example 7

Lutein, an oily component, was prepared. 25 parts by weight of lutein (20% oil suspension preparation) was added to 58 parts by weight of ethanol to obtain a dispersion. 57.5 parts by weight of calcium silicate (trade name "FLORITE R" manufactured by Tokuyama Corporation) was passed through a sieve and filled in a vertical granulator. Then, the beta-carotene dispersion and an aqueous solution obtainable by dissolving 5 parts by weight of apple polyphenol in 144 parts by weight of water were filled and mixed for 5 minutes. Then, 12.5 parts by weight of xylitol was added, and the mixture was subjected to stirring granulation for 5 minutes, sized, and dried. The resulting granule was placed on a tray and allowed to stand at 70°C for 48 hours. The residual ratio of lutein contained in the heated granule was 100.0%.

### Comparative Example 5

Lutein, an oily component, was prepared. 25 parts by weight of lutein (20% oil suspension preparation) was added to 58 parts by weight of ethanol to obtain a dispersion. 57.5 parts by weight of calcium silicate (trade name "FLORITE R" manufactured by Tokuyama Corporation) was passed through a sieve and filled in a vertical granulator. Then, the beta-carotene dispersion and 144 parts by weight of water were filled and mixed for 5 minutes. Then, 17.5 parts by weight of xylitol was added, and the mixture was subjected to stirring granulation for 5 minutes, sized, and dried. The resulting granule was placed on a tray and allowed to stand at 70°C for 48 hours. The residual ratio of lutein contained in the heated granule was 89.0%.

### Comparative Example 6

Lutein (20% oil suspension preparation), an oily component, was placed on a tray and allowed to stand at 70°C for 48 hours. After heating, the residual ratio of lutein was 67.0%.

### Example 8

Lipoic acid, a hardly soluble crystalline powder, was prepared. 20.8 parts by weight of lipoic acid and 2.1 parts by weight of apple polyphenol were added to 40 parts by weight of ethanol to obtain a dispersion. 41.7 parts by weight of calcium silicate (trade name "FLORITE R" manufactured by Tokuyama Corporation) was passed through a sieve and filled in a vertical granulator. Then, the lipoic acid-apple polyphenol dispersion and 125 parts by weight of water were filled and mixed for 5 minutes. Then, 37.5 parts by weight of trehalose was added, and the mixture was subjected to stirring granulation for 5 minutes, sized, and dried. The resulting granule was placed on a tray and allowed to stand at 70°C for 24 hours. The residual ratio of lipoic acid contained in the heated granule was 92.7%.

### Example 9

Lipoic acid, a hardly soluble crystalline powder, was prepared. 20.8 parts by weight of lipoic acid and 4.2 parts by weight of apple polyphenol were added to 40 parts by weight of ethanol to obtain a dispersion. 41.7 parts by weight of calcium silicate (trade name "FLORITE R" manufactured by Tokuyama Corporation) was passed through a sieve and filled in a vertical granulator. Then, the lipoic acid-apple polyphenol dispersion and 125 parts by weight of water were filled and mixed for 5 minutes. Then, 33.3 parts by weight of trehalose was added, and the mixture was subjected to stirring granulation for 5 minutes, sized, and dried. The resulting granule was placed on a tray and allowed to stand at 70°C for 24 hours. The residual ratio of lipoic acid contained in the heated granule was 98.9%.

### Comparative Example 7

Lipoic acid, a hardly soluble crystalline powder, was prepared. 20.8 parts by weight of lipoic acid was added to 40 parts by weight of ethanol to obtain a dispersion. 41.7 parts by weight of calcium silicate (trade name "FLORITE R" manufactured by Tokuyama Corporation) was passed through a sieve and filled in a vertical granulator. Then, the lipoic acid dispersion and 125 parts by weight of water were filled and mixed for 5 minutes. Then, 37.5 parts by weight of trehalose was added, and the mixture was subjected to stirring granulation for 5 minutes, sized, and dried. The resulting granule was placed on a tray and allowed to stand at 70°C for 24 hours. The residual ratio of lipoic acid contained in the heated granule was 75.3%.

### Comparative Example 8

Lipoic acid, a hardly soluble crystalline powder, was placed on a tray and allowed to stand at 70°C for 24 hours. After heating, the residual ratio of lipoic acid was 40.5%.

### Example 10

Vitamin E, an oily component, was prepared. 36.6 parts by weight of vitamin E was added to 20 parts by weight of ethanol to obtain a dispersion. 41.8 parts by weight of calcium silicate (trade name "FLORITE R" manufactured by Tokuyama Corporation) was passed through a sieve and filled in a vertical granulator. Then, the vitamin E dispersion and an aqueous solution obtainable by dissolving 3.7 parts by weight of apple polyphenol in 92 parts by weight of water were filled and mixed for 5 minutes. Then, 17.9 parts by weight of trehalose was added, and the mixture was subjected to stirring granulation for 5 minutes, sized, and dried. The resulting granule was placed on a tray and allowed to stand at 70°C for 72 hours. The residual ratio of vitamin E contained in the heated granule was 100.0%.

### Example 11

Vitamin E, an oily component, was prepared. 36.6 parts by weight of vitamin E was added to 20 parts by weight of ethanol to obtain a dispersion. 41.8 parts by weight of calcium silicate (trade name "FLORITE R" manufactured by Tokuyama Corporation) was passed through a sieve and filled in a vertical granulator. Then, the vitamin E dispersion and an aqueous solution obtainable by dissolving 7.4 parts by weight of apple polyphenol in 92 parts by weight of water were filled and mixed for 5 minutes. Then, 14.2 parts by weight of trehalose was added, and the mixture was subjected to stirring granulation for 5 minutes, sized, and dried. The resulting granule was placed on a tray and allowed to stand at 70°C for 72 hours. The residual ratio of vitamin E contained in the heated granule was 100.0%.

### Comparative Example 9

Vitamin E, an oily component, was prepared. 36.6 parts by weight of vitamin E was added to 20 parts by weight of ethanol to obtain a dispersion. 41.8 parts by weight of calcium silicate (trade name "FLORITE R" manufactured by Tokuyama Corporation) was passed through a sieve and filled in a vertical granulator. Then, the vitamin E dispersion and 92 parts by weight of water were filled and mixed for 5 minutes. Then, 21.6 parts by weight of trehalose was added, and the mixture was subjected to stirring granulation for 5 minutes, sized, and dried. The resulting granule was placed on a tray and allowed to stand at 70°C for 72 hours. The residual ratio of vitamin E contained in the heated granule was 99.5%.

### Comparative Example 10

Vitamin E, an oily component, was placed on a tray and allowed to stand at 70°C for 72 hours. After heating, the residual ratio of Vitamin E was 96.5%.

### Measurement of loose bulk density

An appropriate amount of the granules obtained in the examples were broken up through a No. 16 (1000 µm) sieve. The sample was allowed to flow down into a stainless vessel having a predetermined volume (100 mL) until the sample overflowed. An excess powder deposited in the upper part of the vessel was carefully scraped off with a slide glass or the like. The mass of the powder filled in this vessel was measured, the amount of the powder contained per milliliter was defined as loose bulk density (g/mL). The measurement results are shown in Table 2.

**[Table 2]**

| Example No. | Loose bulk density (g/mL) |
|---|---|
| 1 | 0.45 |
| 2 | 0.41 |
| 3 | 0.42 |
| 4 | 0.37 |
| 5 | 0.38 |
| 6 | 0.38 |
| 7 | 0.40 |
| 8 | 0.36 |
| 9 | 0.35 |
| 10 | 0.43 |
| 11 | 0.44 |
| Comparative Example 1 | 0.42 |
| Comparative Example 3 | 0.38 |
| Comparative Example 5 | 0.40 |
| Comparative Example 7 | 0.37 |
| Comparative Example 9 | 0.44 |

### Production of tablets

5 parts by weight of agar and 1 part by weight of a sucrose fatty acid ester were added to the granules obtained in the examples to obtain a granule for tableting. The granule was tableted using a tableting machine (Model No. HT-AP12SS-U manufactured by Hata Iron Works Co., Ltd.) to obtain tablets. The shapes of the obtained tablets are shown in Table 3.

**[Table 3]**

| Example No. | Functional substance | Shape of tablet |
|---|---|---|
| 2 | Saw palmetto extract | 10-mm circular disc; weight, 400 mg; hardness, 8.0 kgf; and thickness, 5.2 mm |
| 4 | Beta-carotene | 9-mm circular disc; weight, 300 mg; hardness, 7.5 kgf; and thickness, 5.0 mm |
| 6 | Lutein | 9-mm circular disc; weight, 300 mg; hardness, 8.4 kgf; and thickness, 5.0 mm |
| 10 | Vitamin E | 8-mm circular disc; weight, 250 mg; hardness, 9.1 kgf; and thickness, 5.2 mm |

### Apparatuses and conditions

The granulator used in the following examples is a high-speed stirring granulator, Model No. "VG-05" manufactured by Powerex Corporation. For granulation, the rotational speed of a bottom stirrer (blade) is about 300 rpm, and that of a stirrer (cross screw), which rotates in a direction different from that of the bottom stirrer, is about 3000 rpm.

As a drier, a fluidized-bed dryer, Model No. "MP-01" manufactured by Powerex Corporation, is used. The amount of air used for drying was suitably changed depending on the flow condition of the granulation product. The drying air temperature was 75°C, and the granule was dried until the moisture content in the product became 2% or lower. The moisture content was measured based on the loss on drying at 85°C for 15 minutes using an infrared moisture meter manufactured by Mettler K.K

Model No. "COMIL 197S" manufactured by QUADRO K.K. was used for sizing, and a round impeller and a 810-µm screen were used.

As the mold for tablets, Model No. "VIRGO 512HUK" manufactured by Kikusui Sake Co., Ltd. was used, and compression was performed on a turntable at 40 rpm. A metallic mold having a diameter of 10 mm, R15, was used.

Vitamin A derivatives in the sample were extracted using a mixture of an equal amount of isopropyl alcohol and methanol and quantified using a high performance liquid chromatograph equipped with an ultraviolet visible detector using a silica gel bound with an octadecyl group as a filler.

### Example 12

A vitamin A-containing granule was produced as follows.

30 g of vitamin A palmitate ester (1 million units/g) was dissolved in 75 g of a medium-chain fatty acid triglyceride (trade name "COCONAD", a mixture of caprylic acid, capric acid, and triglyceride laurate, manufactured by Kao Corporation). The resulting oily solution was filled in a high-speed stirring granulator together with 100 g of calcium silicate (trade name "FLORITE R" manufactured by Tokuyama Corporation) and stirred. Then, 60 g of xylitol was filled in the granulator, 300 g of purified water in which 12 g of apple polyphenol (trade name "APPLEPHENONE SH" manufactured by Asahi Food and Healthcare Co., Ltd.) was dissolved was added, and granulation was performed. The granulation product was dried using a fluid-bed drier to obtain a vitamin A granule.

After the resulting granule was filled in an aluminum pouch packaging and stored at 70°C for 4 days, the residual ratio of vitamin A was 90%.

### Example 13

A vitamin A-containing granule was produced as follows.
10 g of vitamin A palmitate ester (1 million units/g) was dissolved in 100 g of a medium-chain fatty acid triglyceride (trade name "COCONAD" manufactured by Kao Corporation). The resulting oily solution was filled in a high-speed stirring granulator together with 100 g of calcium silicate (trade name "FLORITE R" manufactured by Tokuyama Corporation) and stirred. Then, 40 g of xylitol and 20 g of hydroxypropylcellulose were filled in the granulator, 200 g of purified water in which 1 g of apple polyphenol (trade name "APPLEPHENONE SH" manufactured by Asahi Food and Healthcare Co., Ltd.) was dissolved was added, and granulation was performed. The granulation product was dried using a fluid-bed drier to obtain a vitamin A granule.

After the resulting granule was filled in an aluminum pouch packaging and stored at 70°C for 4 days, the residual ratio of vitamin A was 80%.

### Example 14

A vitamin A-containing granule was produced as follows. 80 g of vitamin A palmitate ester (1 million units/g) was dissolved in 50 g of a medium-chain fatty acid triglyceride (trade name "COCONAD" manufactured by Kao Corporation). The resulting oily solution was filled in a high-speed stirring granulator together with 100 g of calcium silicate (trade name "FLORITE R" manufactured by Tokuyama Corporation) and stirred. Then, 60 g of xylitol was filled in the granulator, 300 g of purified water in which 50 g of apple polyphenol (trade name "APPLEPHENONE SH" manufactured by Asahi Food and Healthcare Co., Ltd.) was dissolved was added, and granulation was performed. The granulation product was dried using a fluid-bed drier to obtain a vitamin A granule.

After the resulting granule was filled in an aluminum pouch packaging and stored at 70°C for 4 days, the residual ratio of vitamin A was 85%.

### Example 15

A vitamin A-containing granule was produced as follows. 30 g of vitamin A palmitate ester (1 million units/g) was dissolved in 75 g of a medium-chain fatty acid triglyceride (trade name "COCONAD" manufactured by Kao Corporation). The resulting oily solution was filled in a high-speed stirring granulator together with 100 g of calcium silicate (trade name "FLORITE R" manufactured by Tokuyama Corporation) and stirred. Then, 60 g of xylitol was filled in the granulator, 300 g of purified water in which 12 g of apple polyphenol (trade name "APPLEPHENONE SH" manufactured by Asahi Food and Healthcare Co., Ltd.) was dissolved was added, and granulation was performed. The granulation product was dried using a fluid-bed drier. After drying, coating was applied using hydroxypropylmethylcellulose.

After the resulting coated granule was filled in an aluminum pouch packaging and stored at 70°C for 4 days, the residual ratio of vitamin A was 92%.

### Example 16

A vitamin A-containing granule was produced as follows.
30 g of vitamin A palmitate ester (1 million units/g) was dissolved in 75 g of a medium-chain fatty acid triglyceride (trade name "COCONAD" manufactured by Kao Corporation). The resulting oily solution was filled in a high-speed stirring granulator together with 120 g of calcium silicate (trade name "FLORITE R" manufactured by Tokuyama Corporation) and stirred. Then, 50 g of xylitol was filled in the granulator, 280 g of purified water in which 10 g of ascorbic acid was dissolved was added, and granulation was performed. The granulation product was dried using a fluid-bed drier to obtain a vitamin A granule.

After the resulting granule was filled in an aluminum pouch packaging and stored at 70°C for 4 days, the residual ratio of vitamin A was 88%.

### Example 17

A vitamin A-containing granule was produced as follows.
20 g of vitamin A acetate ester (1 million units/g) was dissolved in 80 g of a medium-chain fatty acid triglyceride (trade name "COCONAD" manufactured by Kao Corporation). The resulting oily solution was filled in a high-speed stirring granulator together with 120 g of calcium silicate (trade name "FLORITE R" manufactured by Tokuyama Corporation) and stirred. Then, 50 g of xylitol was filled in the granulator, 300 g of purified water in which 10 g of apple polyphenol (trade name "APPLEPHENONE SH" manufactured by Asahi Food and Healthcare Co., Ltd.) was dissolved was added, and granulation was performed. The granulation product was dried using a fluid-bed drier to obtain a vitamin A granule.

After the resulting granule was filled in an aluminum pouch packaging and stored at 70°C for 4 days, the residual ratio of vitamin A was 85%.

### Example 18

A vitamin A-containing granule was produced as follows. 40 g of vitamin A palmitate ester (1.7 million units/g) was dissolved in 40 g of a medium-chain fatty acid triglyceride (trade name "COCONAD" manufactured by Kao Corporation). The resulting oily solution was filled in a high-speed stirring granulator together with 100 g of calcium silicate (trade name "FLORITE R" manufactured by Tokuyama Corporation) and stirred. Then, 60 g of maltitol and 20 g of hydroxypropylmethylcellulose were filled in the granulator, 300 g of purified water in which 20 g of tea polyphenol (trade name "POLYPHENONE AC" manufactured by Mitsui Norin Co., Ltd.) was dissolved was added, and granulation was performed. The granulation product was dried using a fluid-bed drier to obtain a vitamin A granule.

After the resulting granule was filled in an aluminum pouch packaging and stored at 70°C for 4 days, the residual ratio of vitamin A was 89%.

### Example 19

A vitamin A-containing granule was produced as follows. 20 g of vitamin A palmitate ester (1.7 million units/g) was dissolved in 80 g of a medium-chain fatty acid triglyceride (trade name "COCONAD" manufactured by Kao Corporation). The resulting oily solution was filled in a high-speed stirring granulator together with 100 g of calcium silicate (trade name "FLORITE R" manufactured by Tokuyama Corporation) and stirred. Then, 150 g of xylitol was filled in the granulator, 350 g of purified water in which 120 g of apple polyphenol (trade name "APPLEPHENONE SH" manufactured by Asahi Food and Healthcare Co., Ltd.) was dissolved was added, and granulation was performed. The granulation product was dried using a fluid-bed drier to obtain a vitamin A granule.

After the resulting granule was filled in an aluminum pouch packaging and stored at 70°C for 4 days, the residual ratio of vitamin A was 88%.

### Example 20

A vitamin A granule was obtained in the same manner as in Example 12, except that a medium-chain fatty acid monoglyceride (trade name "HOMOTEX PT" manufactured by Kao Corporation) was used instead of the medium-chain fatty acid triglyceride (trade name "COCONAD" manufactured by Kao Corporation), and storage stability was tested. As a result, the residual ratio of vitamin A was 85%.

### Comparative Example 11

For comparison, a vitamin A-containing granule was produced as follows.
40 g of vitamin A palmitate ester (1 million units/g) was dissolved in 40 g of a medium-chain fatty acid triglyceride (trade name "COCONAD" manufactured by Kao Corporation). The resulting oily solution was filled in a high-speed stirring granulator together with 100 g of calcium silicate (trade name "ELORITE R" manufactured by Tokuyama Corporation) and stirred. Then, 80 g of xylitol was filled in the granulator, 300 g of purified water was added, and granulation was performed. The granulation product was dried using a fluid-bed drier to obtain a vitamin A granule.

After the resulting granule was filled in an aluminum pouch packaging and stored at 70°C for 4 days, the residual ratio of vitamin A was 62%.

### Comparative Example 12

A vitamin A granule was obtained in the same manner as in Comparative Example 11, except that 40 g of vitamin E was used instead of the medium-chain fatty acid triglyceride (trade name "COCONAD" manufactured by Kao Corporation), and storage stability was tested. As a result, the residual ratio of vitamin A was 68%.

### Comparative Example 13

For comparison, a vitamin A-containing granule was produced as follows.
40 g of vitamin A palmitate ester (1 million units/g) was filled in a high-speed stirring granulator together with 100 g of calcium silicate (trade name "FLORITE R" manufactured by Tokuyama Corporation) and stirred. Then, 80 g of xylitol was filled in the granulator, 300 g of purified water in which 20 g of apple polyphenol (trade name "APPLEPHENONE SH" manufactured by Asahi Food and Healthcare Co., Ltd.) was dissolved was added, and granulation was performed. The granulation product was dried using a fluid-bed drier to obtain a vitamin A granule.

After the resulting granule was filled in an aluminum pouch packaging and stored at 70°C for 4 days, the residual ratio of vitamin A was 36%.

### Comparative Example 14

For comparison, a vitamin A-containing granule was produced as follows.
40 g of vitamin A palmitate ester (1 million units/g) was filled in a high-speed stirring granulator together with 100 g of calcium silicate (trade name "FLORITE R" manufactured by Tokuyama Corporation) and stirred. Then, 80 g of xylitol was filled in the granulator, 300 g of purified water was added, and granulation was performed. The granulation product was dried using a fluid-bed drier to obtain a vitamin A granule.

After the resulting granule was filled in an aluminum pouch packaging and stored at 70°C for 4 days, the residual ratio of vitamin A was 19%.

### Comparative Example 15

For comparison, stability of a commercially available vitamin A-containing granule was evaluated.
After a commercially available granule containing vitamin A palmitate ester, trade name "RIKEN DRY A S-200PT" manufactured by Riken Vitamin Co. Ltd. was filled in an aluminum pouch packaging and stored at 70°C for 4 days, the residual ratio of vitamin A was 3%.

### Comparative Example 16

For comparison, stability of a commercially available vitamin A-containing granule was evaluated.
After a commercially available granule containing vitamin A acetate ester, trade name "DRY VITAMIN A ACETATE 325GFP" manufactured by BASF K.K. was filled in an aluminum pouch packaging and stored at 70°C for 4 days, the residual ratio of vitamin A was 27%.

### Measurement of loose bulk density

An appropriate amount of the granules obtained in the above-mentioned examples and the comparative examples were broken up through a No. 16 (1000 µm) sieve. The sample was allowed to flow down into a stainless vessel having a predetermined volume (100 mL) until the sample was overflowed. An excess powder deposited in the upper part of the vessel was carefully scraped off with a slide glass or the like. The mass of the powder filled in this vessel was measured, and the amount of the powder contained per milliliter was defined as loose bulk density (g/mL). The measurement results are shown in Table 4.

**[Table 4]**

| Example | Amount of oily solution (parts)^{a} | Amount of antioxidizing agent (parts)^{a} | Amount of carrier (parts)^{b} | Vitamin A residual ratio (%) | Loose bulk density (g/mL) |
|---|---|---|---|---|---|
| 12 | 250 | 40 | 95.2 | 90 | 0.42 |
| 13 | 1000 | 10 | 90.9 | 80 | 0.36 |
| 14 | 62.5 | 62.5 | 76.9 | 85 | 0.40 |
| 15 | 250 | 40 | 95.2 | 92 | 0.38 |
| 16 | 250 | 33.3 | 114.3 | 88 | 0.24 |
| 17 | 400 | 50 | 120 | 85 | 0.29 |
| 18 | 100 | 50 | 125 | 89 | 0.48 |
| 19 | 400 | 600 | 100 | 88 | 0.68 |
| 20 | 250 | 40 | 95.2 | 85 | 0.42 |
| Comparative Example 11 | 100 | - | 125 | 62 | 0.45 |
| Comparative Example 12 | 100 | - | 125 | 68 | 0.44 |
| Comparative Example 13 | - | 50 | 250 | 36 | 0.48 |
| Comparative Example 14 | - | - | 250 | 19 | 0.42 |
| Comparative Example 15 | - | Unknown | - | 3 | 0.42 |
| Comparative Example 16 | - | Unknown | - | 27 | 0.49 |

| | | | | | |
|---|---|---|---|---|---|
| a) Amount based on 100 parts of vitamin A and analogs b) Amount based on 100 parts of vitamin A-containing liquid | | | | | |

### Example 21

A vitamin A-containing tablet was produced as follows.
890 g of crystalline cellulose and 10 g of magnesium stearate were added to 100 g of the vitamin A-containing granule obtained in Example 12 and mixed to obtain a powder for tableting. The resulting powder for tableting was compressed to mold vitamin A-containing tablets. The shapes of tablets are shown in Table 2.

After the resulting tablets were filled in an aluminum pouch packaging and stored at 70°C for 4 days, the residual ratio of vitamin A was 82%.

### Comparative Example 17

For comparison, a vitamin A-containing tablet was produced as follows.
980 g of crystalline cellulose and 10 g of magnesium stearate were added to 10 g of vitamin A palmitate ester (1 million units/g) and mixed to obtain a powder for tableting. The resulting powder for tableting was compressed to mold vitamin A-containing tablets. The shapes of tablets are shown in Table 2.

After the obtained tablet was filled in an aluminum pouch packaging and stored at 70°C for 4 days, the residual ratio of vitamin A was 46%.

### Example 22

A vitamin A-containing tablet was produced as follows.
590 g of crystalline cellulose, 100 g of shell calcium, 100 g of zinc gluconate, 100 g of magnesium oxide, and 10 g of magnesium stearate were added to 100 g of the vitamin A-containing granule obtained in Example 12 and mixed to obtain a powder for tableting. The resulting powder for tableting was compressed to mold tablets containing vitamin A and minerals. The shapes of tablets are shown in Table 2.

After the resulting tablets were filled in an aluminum pouch packaging and stored at 70°C for 4 days, the residual ratio of vitamin A was 75%.

### Comparative Example 18

For comparison, a vitamin A-containing tablet was produced as follows.
680 g of crystalline cellulose, 100 g of shell calcium, 100 g of zinc gluconate, 100 g of magnesium oxide, and 10 g of magnesium stearate were added to 10 g of vitamin A palmitate ester (1 million units/g) and mixed to obtain a powder for tableting. The resulting powder for tableting was compressed to mold vitamin A-containing tablets. The shapes of tablets are shown in Table 5.

After the resulting tablets were filled in an aluminum pouch packaging and stored at 70°C for 4 days, the residual ratio of vitamin A was 6%.

**[Table 5]**

| Example No. | Shape of tablet |
|---|---|
| 21 | 8.0-mm circular disc; weight, 300 mg; hardness, 25 kgf; and thickness, 5.2 mm |
| Comparative Example 17 | 8.0-mm circular disc; weight, 300 mg; hardness, 24 kgf; and thickness, 5.2 mm |
| 22 | 8.0-mm circular disc; weight, 350 mg; hardness, 19 kgf; and thickness, 4.7 mm |
| Comparative Example 18 | 8.0-mm circular disc; weight, 350 mg; hardness, 18 kgf; and thickness, 4.6 mm |

## Claims

1. A granule comprising:
a carrier comprising porous calcium silicate;
a functional substance which is an oily liquid or a low-melting solid and a water-soluble antioxidizing agent, which are adsorbed on the carrier; and
a granulating component.

2. The granule according to claim 1, wherein the oily liquid or the low-melting solid is a lipophilic substance.

3. The granule according to claim 1 or 2, wherein the water-soluble antioxidizing agent is a polyphenol.

4. The granule according to any one of claims 1 to 3, wherein the water-soluble antioxidizing agent is combined in an amount of 3 to 20 parts by weight based on 100 parts by weight of the functional substance.

5. The granule according to any one of claims 1 to 4, wherein the water-soluble antioxidizing agent is apple polyphenol or hop polyphenol.

6. The granule according to any one of claims 1 to 5, wherein the functional substance is at least one selected from the group consisting of vitamin E, docosahexaenoic acid, eicosapentaenoic acid, lipoic acid, evening primrose oil, beta-carotene and saw palmetto extract.

7. The granule according to any one of claims 1 to 6, wherein the carrier is combined in an amount of 20 to 200 parts by weight based on 100 parts by weight of the functional substance, the granulating component is combined in an amount of 30 to 300 parts by weight based on 100 parts by weight of the carrier, and the granule has a loose bulk density of 0.20 to 0.80 g/ml.

8. A granule comprising:
porous calcium silicate;
a vitamin A-containing liquid which is obtained by dissolving or dispersing vitamin A or a vitamin A derivative in an oily liquid or a low-melting solid;
a water-soluble antioxidizing agent; and
a granulating component.

9. The granule according to claim 8, wherein the oily liquid or the low-melting solid is a fatty acid glyceride.

10. The granule according to claim 8 or 9, wherein the oily liquid or the low-melting solid is contained in an amount of 50 to 1200 parts by weight based on 100 parts by weight of the vitamin A or the vitamin A derivative.

11. The granule according to any one of claims 8 to 10, wherein the water-soluble antioxidizing agent is apple polyphenol, hop polyphenol, tea polyphenol or vitamin C.

12. The granule according to any one of claims 8 to 11, wherein the vitamin A derivative is palmitate ester or acetate ester of vitamin A.

13. The granule according to any one of claims 8 to 12, wherein the water-soluble antioxidizing agent is contained in an amount of 5 to 800 parts by weight based on 100 parts by weight of the vitamin A or the vitamin A derivative.

14. The granule according to any one of claims 8 to 13, wherein the porous calcium silicate is contained in an amount of 50 to 500 parts by weight based on 100 parts by weight of the vitamin A-containing liquid, the granulating component is contained in an amount of 30 to 500 parts by weight based on 100 parts by weight of the porous calcium silicate, and the granule has a loose bulk density of 0.20 to 0.80 g/ml.

15. The granule according to any one of claims 1 to 14, coated with a water-soluble polymer.

16. The granule according to any one of claims 1 to 15, wherein the granulating component is at least one selected from the group consisting of starches and sugars.

17. A method for producing a granule comprising the steps of:
adsorbing a functional substance and polyphenol on a carrier comprising porous calcium silicate; and
mixing the resulting supported material with a granulating component to perform granulation.

18. A method for producing a granule comprising the steps of:
dissolving or dispersing vitamin A or a vitamin A derivative in an oily liquid or a low-melting solid to obtain a vitamin A-containing liquid;
mixing the vitamin A-containing liquid and porous calcium silicate;
and
mixing the resulting mixture, a granulating component and a water-soluble antioxidizing agent.

19. A method for producing a tablet comprising the steps of:
charging the granule according to any one of claims 1 to 16 into a cylindrical mortar; and
compressing the granule filled in the mortar with a pestle.

20. A tablet obtainable by the method according to claim 19.
